# EUROPEAN PATENT SPECIFICATION

(11) **EP 1 765 250 B1**
(45) Date of publication and mention of the grant of the patent: **05.09.2018**
(21) Application number: 05760379.7
(22) Date of filing: 09.06.2005
(51) Int. Cl.: A61F 13/511, A61F 13/537

(54) **TOPOGRAPHICAL COMPOSITE LINERS**
TOPOGRAPHISCHES KOMPOSIT-MATERIAL
REVÊTEMENTS COMPOSITES TOPOGRAPHIQUES

(30) Priority: 29.06.2004 US 882914
(43) Date of publication of application: 28.03.2007
(73) Proprietor: KIMBERLY-CLARK WORLDWIDE, INC., Neenah, WI 54956 (US)
(72) Inventor: ELLINGSON, Alissa, R., Appleton, Wisconsin 54915 (US); COHEN, Jason, C., Appleton, Wisconsin 54915 (US); BARATIAN, Stephen, A., Roswell, Georgia 30076 (US); GOSAIN, Kusum, Appleton, Wisconsin 54915 (US); TAILOR, Vipula, J., Alpharetta, Georgia 30004 (US); BUHROW, Chantel, S., Weyauwega, Wisconsin 54983 (US)
(74) Representative: Dehns
(86) International application number: PCT/US2005/020290
(87) International publication number: WO 2006/007340

(56) References cited:
- EP-A- 1 022 007
- WO-A1-01/26595
- WO-A1-91/11161
- PATENT ABSTRACTS OF JAPAN vol. 2003, no. 11, 5 November 2003 (2003-11-05) & JP 2003 204993 A (KAO CORP), 22 July 2003 (2003-07-22)

## Description

### Background

The present invention relates to composite liners. More particularly, the present invention relates to composite liners suitable for incorporation into disposable absorbent articles.

Disposable absorbent articles, including diapers, training pants, incontinence devices, menstrual pads and the like, are designed to absorb and contain body exudates and are generally single-use or disposable. Such products usually are placed against or in proximity to the wearer's body to absorb and contain various exudates discharged from the body. These products typically include a fluid pervious bodyside liner or cover, a liquid impermeable outer cover or backsheet, and an absorbent structure disposed between the bodyside liner and outer cover. The absorbent article may include a surge layer subjacent to and in liquid communicating contact with the bodyside liner. The surge layer may also be subjacent to and in liquid communicating contact with the absorbent structure. The absorbent structure may include an absorbent core often formed of a blend or mixture of cellulosic pulp fluff fibers and absorbent gelling particles.

Conventional bodyside liner materials are liquid pervious layers constructed of a nonwoven fabric such as a layer of polypropylene spunbond fibers. Bodyside liners are designed to provide a liquid pervious barrier between a wearer of a personal care absorbent article and any absorbent structures beneath the liner. With this in mind, it is known to provide bodyside liners which are liquid pervious and that do not retain liquids. Such liners merely act as a pass through or separation layer. The structure of such bodyside liners is optimized primarily based on providing liquid intake and dryness, mostly with respect to urine.

A highly desired characteristic of disposable absorbent articles is the ability to collect and retain urine, menses and fecal material deposited thereon by the wearer. Dealing with fecal material and menses collected by the disposable absorbent article is simply more difficult than dealing with urine, due to the complex rheology of low-viscosity fecal material and menses.
Attempts to deal with fecal material include providing a topsheet that conforms closely to the wearer and has an aperture. The aperture is intended to be registered with the anal opening, so that fecal material passes through the aperture into a void space. The topsheet may comprise various elastic panels in order to closely conform to the skin of the wearer, and/or may have linear elastic strands. Improvements have been made in this area, such as optimizing the material properties of the topsheet. Such optimization makes the topsheet more comfortable to the wearer and allows a single disposable absorbent article to fit a larger range of sizes of wearers.

Improvements to this genre of disposable absorbent articles also include the addition of spacers. Spacers may be interposed between the topsheet and the core, in order to ensure a void space is present to receive the fecal material.

Still other attempts provide barrier leg cuffs that are upstanding from the plane of the topsheet. The barrier leg cuffs prevent fecal material from breaching the perimeter of the disposable absorbent article.

However, none of these attempts to handle fecal material solves the problem of low-viscosity fecal material that is prevalent in younger children, particularly those who are breast fed. Low-viscosity fecal material easily migrates within the disposable absorbent article under the influences of gravity and motion or pressure by the wearer.

The migration of the fecal material often moves it towards the perimeter of the disposable absorbent article, increasing the likelihood of leakage. The migration of the fecal material also smears it against the skin of the wearer, making cleanup more difficult. In order to clean the wearer, the caretaker must wipe the entire area of the skin that has encountered the fecal material and typically has to deal with a relatively large soiled area.

Accordingly, there exists a need to provide a composite liner that provides improved intake of low viscosity fecal material. Further, there exists a need to minimize the amount of low-viscosity fecal material remaining on the skin of the wearer once a disposable absorbent article is removed. Prior art can be found in WO-A-01/26595.

### Summary

In response to the foregoing need, the present inventors undertook intensive research and development efforts that resulted in the discovery of a liner composite. One version of the liner composite of the present invention includes a fluid pervious liner having a bodyfacing surface and a lower surface. The liner composite also includes a topographical surge material having a bodyfacing surface, and a plurality of topographical features where at least a portion of the bodyfacing surface of the topographical surge material in facing relationship with at least a portion of the lower surface of the fluid pervious liner. Additionally, at least a portion of the topographical features of the topographical surge material are imparted onto the bodyfacing surface of the fluid pervious liner, and a plurality of the portions of the topographical features imparted onto the bodyfacing surface of the fluid pervious liner have an amplitude of greater than 2.5 mm.

Another version of the present invention provides a disposable absorbent article, the article including a liquid impervious backing sheet, and a fluid pervious liner having a bodyfacing surface and a lower surface. The article also including an absorbent core disposed between the fluid pervious bodyside liner and the liquid impervious backing sheet; and a topographical surge material having a bodyfacing surface, and a plurality of topographical features, the topographical surge material disposed between the absorbent core and the fluid pervious bodyside liner. Additionally, at least a portion of the topographical features of the topographical surge material are imparted onto the bodyfacing surface of the fluid pervious liner, and a plurality of the portions of the topographical features imparted onto the bodyfacing surface of the fluid pervious liner have an amplitude of greater than 2.5 mm.

It is to be understood that both the foregoing general description and the following detailed description are exemplary and are intended to provide further explanation of the invention claimed. The accompanying drawings, that are incorporated in and constitute part of this specification, are included to illustrate and provide further understanding of the articles of the invention. Together with the description, the drawings serve to explain various aspects of the invention.

### Drawings

The foregoing and other features, aspects and advantages of the present invention will become better understood with regard to the following description, appended claims and accompanying drawings where:
FIGs. 1 and 2 illustrate cross sectional views of versions of a liner composite;
FIGs. 3 through 6 illustrate several cross sectional views of configurations of a topographical surge;
FIG. 7 illustrates a cross sectional view of a version of the liner composite;
FIG. 8 illustrates an enlarged cross sectional view of a topographical surface;
FIG. 9 representatively illustrates a partially cut-away, top plan view of the bodyfacing or upper surface of a disposable absorbent article incorporating a version of the liner composite.

### Detailed Description of the Invention

The present disclosure of the invention will be expressed in terms of its various components, elements, constructions, configurations, arrangements and other features that may also be individually or collectively be referenced by the term, "aspect(s)" of the invention, or other similar terms. It is contemplated that the various forms of the disclosed invention may incorporate one or more of its various features and aspects, and that such features and aspects may be employed in any desired, operative combination thereof.

It should also be noted that, when employed in the present disclosure, the terms "comprises", "comprising" and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.

The present invention is directed to solving problems related to liner composites including leakage of fecal material and menses. Additionally, the present invention is directed to liner composites which minimize the amount of low-viscosity fecal material remaining on the skin of the wearer once a disposable absorbent article is removed. This detailed description of the present invention will include a description of a representative absorbent article including the various components of such articles. The description of the representative absorbent article will also include a description of the features encompassed by the present invention.

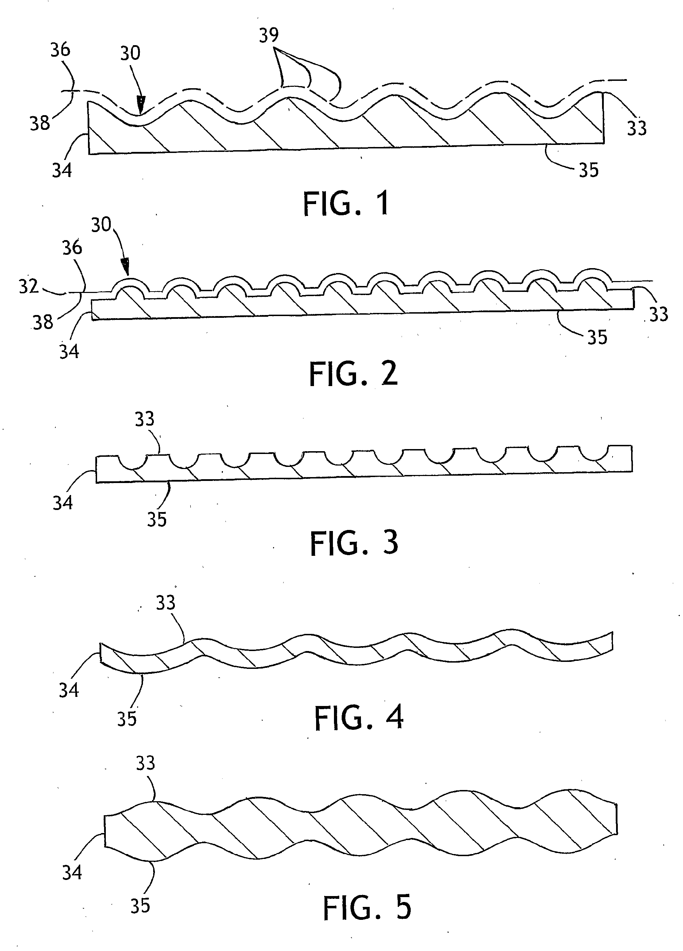

As illustrated in FIGs. 1, 2, and 7, the liner composite (30) of the present invention includes a sheet of fluid pervious liner material (32) and a topographical surge (34). The liner material (32) has a bodyfacing or upper surface (36) and an opposing or lower surface (38). The topographical surge (34) has a bodyfacing or upper surface (33) and an opposing or lower surface (35). The liner material (32) and the topographical surge (34) are in liquid communication with one another. As used herein when describing the liner material (32) in a relation to the topographical surge (34) and vice versa, the term "liquid communication" means that liquid is able to travel from one layer to another layer or one location to another location. The liner material (32) may be attached to the topographical surge (34) by adhesive, cohesive, pressure, thermal or ultrasonic bonds either directly or indirectly.

The liner material (32) is suitably fluid pervious. When incorporated into a disposable absorbent article, the liner material (32) is in close proximity to the skin of the wearer. Consequently, the liner material (32) is desirably as compliant, soft feeling, and non-irritating to the wearer's skin as possible.

A suitable liner material (32) may be manufactured from a wide range of materials including, but not limited to woven and nonwoven materials, apertured formed thermoplastic films, apertured plastic films, hydro-formed films, porous foams, reticulated foams, reticulated thermoplastic films, and thermoplastic scrims. Suitable woven and nonwoven materials can include natural fibers (e.g., wood or cotton fibers), synthetic or modified natural fibers (e.g., polymeric fibers, such as polyester, polypropylene fibers, and polyethylene, or polyvinylalcohol, starch base resins, polyurethanes, cellulose esters, nylon, and rayon fibers), or a combination of natural and synthetic fibers. When the liner material (32) includes a nonwoven web, the web may be spunbond, carded, wet-laid, meltblown, hydroentangled, combinations of the above, or the like. The liner material (32) is suitably employed to help isolate the wearer's skin from liquids. The liner material (32) can also be made from extensible materials as are described in U.S. Patent No. 6,552,245 issued on April 22, 2003 to Roessler et al. The liner material (32) can also be made from biaxially stretchable materials as are described in WO 02/34184 filed on October 27, 2000 by Vukos et al.

The liner material (32) may be composed of a substantially hydrophobic material, and the hydrophobic material may optionally be treated with a surfactant or otherwise processed to impart a desired level of wettability and hydrophilicity. In a particular embodiment of the present invention, the liner material (32) is made from a nonwoven, spunbond, polypropylene fabric composed of fibers having a fiber diameter of about 21 to 23 microns formed into a web having a basis weight of about 20 grams per square meter and a density of about 0.13 grams per cubic centimeter. The fabric may be surface treated with about 0.3 weight percent of a surfactant, such as a surfactant commercially available from Hodgson Textile Chemicals, Inc. under the trade designation AHCOVEL Base N-62. The surfactant may be applied by any conventional means, such as spraying, printing, foaming, brush coating or similar techniques. The surfactant may be applied to the entire liner material (32) or may be selectively applied to particular sections of the liner material (32), such as the medial section, to provide greater wettability of such sections. The liner material (32) may further include a lotion or treatment applied thereto that is configured to be transferred to the wearer's skin. Suitable compositions for application to the liner material (32) are described in U.S. Patent No. 6,149,934 that issued to Krzysik et al. on November 21, 2000.

Desirably the caliper of the liner material (32) is less than about 6 mm, alternatively, less than about 4 mm, and finally, alternatively, less than about 2 mm; although the approximate caliper may vary according to, *inter alia,* the general design and intended use of the liner material (32).

As shown in FIG. 1, the liner material (32) may include apertures (39). The apertures (39) may be randomly or uniformly arranged through the liner material (32). Alternatively, the apertures (39) can be selectively confined to certain areas of the liner material (32), e.g., located in a narrow longitudinal band or strip within the liner material (32). The size, shape and number of apertures (39) can be varied depending on the desired application. The apertures (39) may be formed in the liner material (32) by any suitable method, for example, by pin aperturing, laser perforation, hydraulic rearrangement, slitting and stretching of the polymeric film, or vacuum aperturing, wherein the resulting apertured cover has an open area and a plurality of protuberances. The protuberances may have a tapered profile.

The number of apertures (39) per square inch may range from about 6 apertures/in² (1 aperture/cm²) to about 1100 apertures/in² (170 apertures/cm²) and preferably ranges from about 50 apertures/in² (8 apertures/cm²) to about 300 apertures/in² (46 apertures/cm²). The apertures may be circular in shape; alternatively, the apertures may be oval in shape or any other shape. The dimensions of the apertures may be uniform, all the apertures having the same size; alternatively, the apertures may have sizes that vary from one aperture to another, or one set of apertures to another set of apertures. Desirably the apertures have one dimension that is greater than about 0.25 mm, alternatively greater than about 0.5 mm, alternatively, greater than about 1 mm, and finally, alternatively, greater than about 2 mm, although the approximate dimension may vary according to, *inter alia,* the general design and intended use of the liner composite (30).

The apertures may be uniformly, non-uniformly or randomly disposed over the full surface of over a portion of the liner material (32). In particular embodiments, the apertures are disposed in a predetermined portion of the liner material (32) which may define a rectangular or oblong area and may be centrally located on the surface of the liner material (32). Alternatively, the apertures may cover the entire area of the liner material (32). The apertures in the liner material (32) may coincide with a portion of the liner that overlays the topographical surge (34). The apertures may allow a fluid impinging upon the outer surface of the liner material (32) to be quickly transferred through the liner material (32).

Alternatively, the liner material (32) may be coapertured with the topographical surge (34) or any other material. The term "coapertured" refers to a composite wherein at least two materials are apertured together to create holes which extend through the layers.

Various woven and nonwoven fabrics can be used to construct the topographical surge (34). For example, the topographical surge (34) may be a layer composed of a meltblown or spunbond web of synthetic fibers, such as polyolefin fibers. The topographical surge (34) may also be a bonded-carded-web or an airlaid web composed of natural and synthetic fibers. The bonded-carded-web may, for example, be a thermally bonded web that is bonded using low melt binder fibers, powder or adhesive. The webs can optionally include a mixture of different fibers. The topographical surge (34) may be composed of a substantially hydrophobic material, and the hydrophobic material may optionally be treated with a surfactant or otherwise processed to impart a desired level of wettability and hydrophilicity. Particular embodiments of the topographical surge (34) includes a hydrophobic, fibrous nonwoven material having a basis weight of greater than about 30 gsm, alternatively, greater than about 50 gsm, and finally, alternatively, greater than about 70 gsm. Another embodiment of the topographical surge (34) includes a nonwoven, bonded-carded-web comprising polyethylene/polyester bicomponent fibers, the web having a basis weight within the range of about 17 - 102 gsm and a density within the range of about 0.02 - 1.0 gm/cc, and the fibers having a size within the range of 0.9 - 18 denier.

Desirably, a surge material can rapidly accept and temporarily hold the liquid prior to releasing the liquid into the storage or retention portions of the absorbent structure. Examples of suitable surge materials are described in U.S. Patent No. 5,486,166 issued to Ellis et al.*,* and U.S. Patent No. 5,490,846, issued to Ellis et al. The topographical surge (34) is typically less hydrophilic than the absorbent core which it may be associated with, and has an operable level of density and basis weight to quickly collect and temporarily hold liquid surges, to transport the liquid from its initial entrance point and to substantially completely release the liquid to other parts of the absorbent core. This configuration can help prevent the liquid from pooling and collecting on the portion of the liner material (32) positioned against the wearer's skin, thereby reducing the feeling of wetness by the wearer. The structure of the topographical surge (34) also generally enhances the air exchange within an absorbent article.

The term "topographical" refers to a surface that does not exist in a single plain, for example a surface that contains portions that are either raised or lowered The topography may be imparted to the topographical surge (34) in numerous ways. For example, the web that forms the topographical surge (34) may be formed on a surface that includes topographical features. The web may then be bonded such as with hot air to provide a fabric with surface features. Alternatively, the web that forms the topographical surge (34) may be creped or otherwise mechanically strained to provide topographical features. The web may also be patterned bonded thereby providing higher and lower densities in the web which may impart a topography to the web. The topographical surge (34) may be formed by a differential basis weight. This differential basis weight may be accomplished by either adding or removing material from the web either before or after the web is bonded into a fabric.

As illustrated in FIGs. 1 - 8, the bodyfacing surface (33) of the topographical surge (34) may take on numerous surface topographies. Additionally, the lower surface (35) of the topographical surge (34) may take on numerous surface topographies. FIG. 1 illustrates a liner composite (30) with a topographical surge (34) that has a generally flat lower surface (35) and a topographical bodyfacing surface (33). Specifically the bodyfacing surface (33) has a topography similar to that of a sine wave with generally uniform amplitude and wavelength. This surface topography may exist in only the lateral or longitudinal direction of the topographical surge (34) in which case the surface topography may resemble corrugations. This surface topography may exist in both the lateral and longitudinal direction of the topographical surge (34) in which case the surface topography may resemble a quilted pattern.

FIG. 2 illustrates another topographical surge (34) that has a generally flat lower surface (35) and a topographical bodyfacing surface (33). Specifically the bodyfacing surface (33) has a topography which resembles a flat surface with semi circles protruding upwards from the flat surface. The semi circles are of uniform size, shape and spacing. Alternatively, the size, shape and spacing may be non-uniform. FIG. 3 illustrates another topographical surge (34) that has a generally flat lower surface (35) and a topographical bodyfacing surface (33). Specifically the bodyfacing surface (33) has a topography which resembles a flat surface with semi circles protruding downward into the flat surface. The semi circles are of uniform size, shape and spacing. Alternatively, as described above, the size, shape and spacing may be non-uniform.

FIG. 4 illustrates a topographical surge (34) that has a topographical lower surface (35) and a topographical bodyfacing surface (33). Specifically both the bodyfacing surface (33) and the lower surface (35) have a topography that resembles sine waves. More specifically, the sine waves of the bodyfacing surface (33) and the lower surface (35) are in phase such that the thickness along the length of the topographical surge (34) generally is uniform. FIG. 5 illustrates a topographical surge (34) that has a topographical lower surface (35) and a topographical bodyfacing surface (33). Specifically both the bodyfacing surface (33) and the lower surface (35) have a topography that resembles a sine wave. More specifically, the sine waves of the bodyfacing surface (33) and the lower surface (35) are 180 degrees out of phase such that the thickness along the length of the topographical surge (34) is at a maximum when the surface of the bodyfacing surface (33) is at a peak, and the thickness of the topographical surge (34) is at a minimum when bodyfacing surface is at a trough.

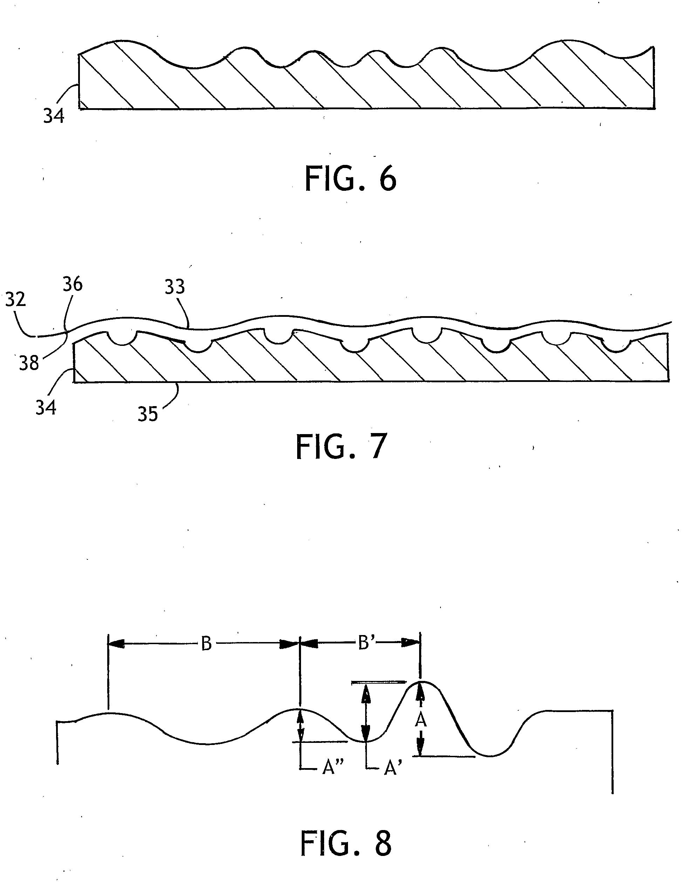

FIG. 6 illustrates another topographical surge (34). Specifically the bodyfacing surface (33) has a topography similar to that of a sign wave with uniform amplitude; however, the wavelength varies from longer wavelengths to shorter wavelengths. Additionally, the amplitude may vary, for example in a pattern of low, high, low, high, etc. or in a pattern from a minimum to a maximum and then back to a minimum.

FIG. 7 illustrates another topographical surge (34) that has a flat lower surface (35) and a topographical bodyfacing surface (33). Specifically the bodyfacing surface (33) has a topography similar to that of a sine wave with uniform amplitude and wavelength, in addition, the topography contain semi circles that protrude downward into the surface, at locations where the sine wave is at a peak and where the sine wave is at a minimum. This is one example where the topography may contain multiple elements or combinations of elements. Specifically, elements of a sine wave combined with elements of downward facing semi circles.

These examples illustrate how surface features of the topographical surge (34) may be combined and modified. Additionally, while the surface features have been described as viewed from a cross sectional perspective, one skilled in the art will recognize that in a given topographical surge (34) the topographical features may differ when comparing the pattern in a longitudinal direction versus the pattern in a lateral direction. For example, the amplitude of the topographical features may be uniform in a longitudinal direction, but may vary in a lateral direction.

The liner material (32) desirably has an appropriate amount of flexibility, or drape, such that the when the liner material (32) and the topographical surge (34) come together to form the liner composite (30), the topography of the topographical surge (34) is at least partially imparted on the bodyfacing surface (36) of the liner material (32).

As illustrated in FIGs. 1, 2, and 7, the topography of the topographical surge (34) is imparted on the bodyfacing surface (36) of the liner material (32). This may take place in several different forms. As shown in FIG. 1, all topographical features of the topographical surge (34) may be imparted on the bodyfacing surface (36) of the liner material (32) such that the shape of the bodyfacing surfaces (33, 36) of the liner material (32) and the topographical surge (34) are effectively identical.

Alternatively, as illustrated in FIGs. 2 and 7, the topographical surge (34) may contain surface features that are not completely imparted on the bodyfacing surface (36) of the liner material (32). The drapability of the liner material (32) and the size and shape of the surface features of the topographical surge (34) may be such that the liner material (32) does not contour to each and every aspect of the surface features of the topographical surge (34), therefore only a portion of the topographical features of the topographical surge (34) are imparted on the bodyfacing surface (36) of the liner material (32). The resulting liner composite (30) therefore has pockets that exist between the liner material (32) and the topographical surge (34). These pockets may provide for storage and distribution of insulting exudates while providing separation from the skin of the wearer.

Providing the surface of the liner composite (30) and the topography of the surface of the liner composite (30) with separate materials provides unique benefits. For example, a first material may have a high degree of resiliency, but may be undesirable for skin contact. This first material may form the topographical surge (34) while a soft, skin friendly material, which may have very little resiliency, may form the liner material (32). Secondly, in many applications the topography is desired in one area of the liner composite (30) and not in another. For example, the topography may be desired in the area of the liner composite (30) which is likely to receive an insult, while for cost, aesthetics, or functional reasons, no topography may be desired in the rest of the liner composite (30). Additionally, for aesthetic and manufacturing purposes, a uniform liner is desired. The liner composite (30) makes it possible to satisfy these two desires. Further, the liner composite (30) may contain a first topographical surge (34) and a second, the first topographical surge (34) may be designed for accepting feces and located accordingly, while the second surge may be designed for accepting urine and also located accordingly. The first and second topographical surges may have similar topographies, or alternatively, the first and second topographical surges may have dissimilar topographies. Further, the liner composite (30) may contain a first topographical surge (34) and a second surge which does not have any surface topography. Further still, the two surge materials may differ in basis weight, density or composition.

The topographical features of the liner composite (30) are desirably resilient and can minimize contact of a wearer's skin with the liner material (32). It is believed that the "land" areas or recesses between topographical features allow for runny bowel movement to reside in these lower areas and away from a wearer's skin. The topographical features may also deter the movement of feces across the bodyfacing surface (36) of the liner material (32) thus minimizing the spread of fecal matter. This is advantageous because it may provide for a smaller spreading pattern and minimize the contact area of the fecal matter with the skin of a wearer of the absorbent article. Desirably, the bodyfacing surface (36) of the liner composite (30) has projections and optional depressions or other structures that are compressible and return to the original shapes to provide separation between a wearer and the liner composite (30).

FIG. 8 illustrates two measurements than can be used to characterize topographical features. The first measurement, wavelength, as shown as B and B', is the distance, in a horizontal direction, between a peak and an adjacent peak, or alternatively a trough and an adjacent trough. As illustrated, the wavelength measured from a first peak to a second peak (B) may be different from the wavelength measured from the first peak to a third peak (B'). The second measurement, amplitude, as shown as A, A', and A", is the distance, in a vertical direction, between a peak and an adjacent trough. As illustrated, the amplitude measured from a first peak to a first trough (A) may be different from the amplitude measured from the first peak to a second trough (A').

Desirably the wavelength of the surface features on the bodyfacing surface (36) of the liner material (32) is between about 5 mm and about 25 mm, alternatively, between about 10 mm and about 20 mm, and finally, alternatively, between about 12 mm and about 16 mm, although the approximate dimension may vary according to, *inter alia,* the general design and intended use of the liner composite (30).

Desirably the amplitude of the topographical features imparted onto the bodyfacing surface (36) of the liner material (32) is greater than about 2 mm, alternatively greater than about 2.5 mm, alternatively, greater than about 5 mm, alternatively, greater than about 15 mm, and finally, alternatively, greater than about 20 mm. In a give embodiment, the amplitude of the surface features on the bodyfacing surface (36) of the liner material (32) is from about 2 mm to about 10 mm. In another embodiment, the amplitude of the surface features on the bodyfacing surface (36) of the liner material (32) is from about 5 mm to about 20 mm, although the approximate amplitude may vary according to, *inter alia,* the general design and intended use of the liner composite (30).

Desirably the ratio of the area of the topographical surge (34) to the area of the liner material (32) is no greater than about 1.00, alternatively no greater than 0.75, alternatively, no greater than about 0.50, alternatively, no greater than about .25, and finally, alternatively, no greater than about 0.10; although the approximate ratio may vary according to, *inter alia,* the general design and intended use of the liner composite (30).

The various aspects, benefits, and versions of the liner composite (30) will be described in the context of a disposable absorbent article, such as a disposable diaper. It is, however, readily apparent that one or more versions of the liner composite (30) could also be employed with other disposable absorbent articles, such as feminine hygiene articles, children's training pants, adult incontinence garments, bandages, bed pads, health care devices and the like. For example, the liner composites (30) of the present invention may be incorporated into disposable diapers similar to those described in U.S. Patent No. 5,509,915, issued to Hanson et al.*,* and U.S. Patent No. 5,192,606, issued to Proxmire et al.

Typically, disposable absorbent articles are intended for limited use and are not intended to be laundered or otherwise cleaned for reuse. A disposable diaper, for example, is discarded after it has become soiled by the wearer. Optionally, a disposable diaper may include a single-use, absorbent insert, and a limited-use outer cover which may be reused several times.

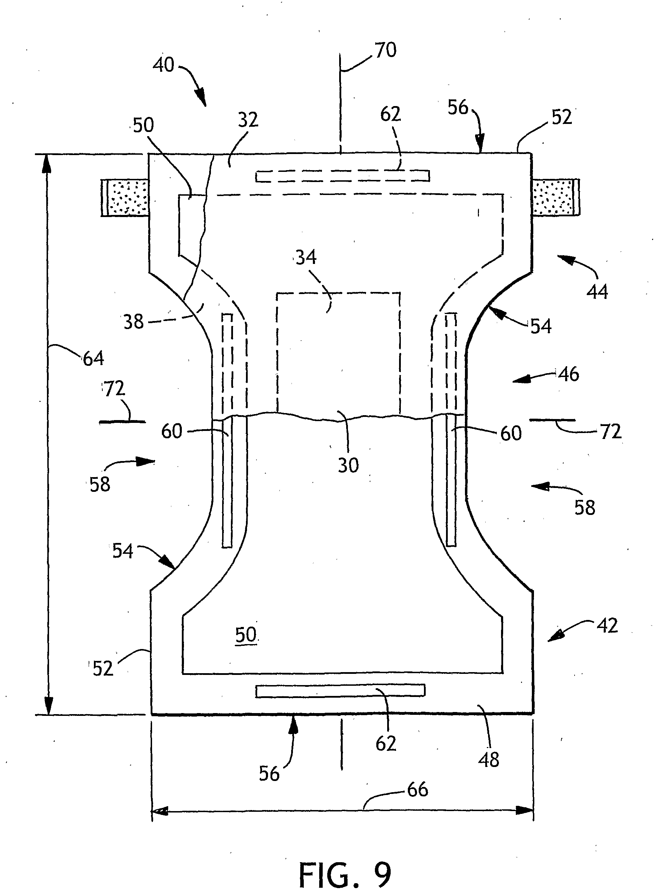

FIG. 9 illustrates a disposable diaper (40) as having a front portion (42), a rear portion (44), and a crotch portion (46) located between the front and rear portions. The disposable diaper includes an outer cover (48), a liner composite (30), and an absorbent core (50) situated between the outer cover (48) and the liner composite (30). The outer edges of the diaper (40) define a periphery (52) with laterally opposed, longitudinally extending side edges (54); longitudinally opposed, laterally extending end edges (56); and a system of elastomeric gathering members, such as a system including leg elastics (60) and waist elastics (62). The longitudinal side edges (54) define leg openings (58) for the diaper (40), and optionally, are curvilinear and contoured. The lateral end edges (56) are illustrated as straight, but optionally, may be curvilinear. The diaper (40) additionally has a longitudinal centerline (70) and a lateral centerline (72). The diaper (40) may also include additional components to assist in the acquisition, distribution and storage of bodily waste. For example, the diaper (40) may include a transport layer, such as described in U.S. Patent No. 4,798,603, issued to Meyer et al. This transport layer may comprise the topographical surge (34) of the composite liner (30); alternatively, this transport layer may be separate from the topographical surge (34) of the composite liner (30).

With regard to the designated surfaces of the absorbent article and its components, the various upper or bodyfacing surfaces are configured to face toward the body of the wearer when the absorbent article is worn by the wearer for ordinary use. The various opposing or lower surfaces are configured to face away from the wearer's body when the absorbent article is worn by the wearer.

The diaper (40) generally defines a longitudinally extending length dimension (64), and a laterally extending width dimension (66), as representatively illustrated in FIG. 9. The diaper may have any desired shape, such as rectangular, I-shaped, a generally hourglass shape, or a T-shape.

The outer cover (48) and the liner composite (30) may be generally coextensive (e.g., FIG. 9), or optionally, may be non-coextensive. Either or both of the outer cover (48) and the liner composite (30) may have length and width dimensions which are generally larger than those of the absorbent core (50) and extend beyond the corresponding dimensions of the absorbent core (50) to provide longitudinal side edges (54) and lateral end edges (56) which may be connected or otherwise associated together in an operable manner. As used herein when describing the liner composite (30) in relation to the outer cover (48) and vice versa, the term "associated" encompasses configurations in which the liner composite (30) is directly joined to the outer cover (48), and configurations where the liner composite (30) is indirectly joined to the outer cover (48) by affixing portions of the liner composite (30) to intermediate members which in turn are affixed to at least portions of the outer cover (48). The liner composite (30) and the outer cover (48) can, for example, be joined to each other in at least a portion of the diaper periphery (52) by attachment mechanisms (not shown) such as adhesive bonds, sonic bonds, pressure bonds, thermal bonds, pinning, stitching, or a variety of other attachment techniques known in the art, as well as combinations thereof.

The outer cover (48) may suitably be composed of a material which is either liquid permeable or liquid impermeable. It is generally desirable that the outer cover (48) be formed from a material which is substantially liquid impermeable. For example, a typical outer cover (48) can be manufactured from a thin plastic film or other flexible liquid impermeable material. For example, the outer cover (48) may be formed from a polyethylene film having a thickness of from about 0.012 mm (0.5 mil) to about 0.051 mm (2.0 mils). If desirous of presenting the outer cover (48) with a more cloth-like feel, the outer cover (48) may include a polyethylene film laminated to the lower or opposing surface thereof a nonwoven web, such as a spunbond web of polyolefin fibers. For example, a polyethylene film having a thickness of about 0.015 mm (0.6 mil) may have thermally laminated thereto a spunbond web of polyolefin fibers, which fibers have a thickness of about 1.5 to about 2.5 denier per filament, which nonwoven web has a basis weight of about 24 gsm (0.7 osy). Methods of forming such cloth-like outer covers are known to those skilled in the art.

Further, the outer cover (48) may be formed of a woven or nonwoven fibrous web layer which has been totally or partially constructed or treated to impart a desired level of liquid impermeability to selected regions that are adjacent or proximate the absorbent core (50). Still further, the outer cover (48) may optionally be composed of micro-porous "breathable" material which permits vapors to escape from the absorbent core (50) while still preventing liquid exudates from passing through the outer cover (48).

The absorbent core (50) may include a matrix of hydrophilic fibers, such as a web of cellulosic fluff, mixed with particles of a high-absorbency material commonly known as superabsorbent material. In a particular version, the absorbent core (50) includes a mixture of superabsorbent hydrogel-forming particles and wood pulp fluff. The wood pulp fluff may be exchanged with synthetic polymeric, meltblown fibers or with a combination of meltblown fibers and natural fibers. The superabsorbent particles may be substantially homogeneously mixed with the hydrophilic fibers or may be non-uniformly mixed.

The absorbent core (50) may have any of a number of shapes. For example, the absorbent core (50) may be rectangular, I-shaped or T-shaped. It is generally desired that the absorbent core (50) be narrower in the crotch portion than the rear or front portion(s).

The high-absorbency material can be selected from natural, synthetic and modified natural polymers and materials. The high-absorbency materials can be inorganic materials, such as silica gels, or organic compounds, such as crosslinked polymers. The term "crosslinked" refers to any means for effectively rendering normally water-soluble materials substantially water insoluble, but swellable. Such means can include, for example, physical entanglement, crystalline domains, covalent bonds, ionic complexes and associations, hydrophilic associations, such as hydrogen bonding, and hydrophobic associations or Van der Waals forces.

Examples of synthetic, polymeric, high-absorbency materials include the alkali metal and ammonium salts of poly(acrylic acid) and poly(methacrylic acid), poly(acrylamides), poly(vinyl ethers), maleic anhydride copolymers with vinyl ethers and alpha-olefins, poly(vinyl pyrolidone), poly(vinyl morpholinone), poly(vinyl alcohol), and mixtures and copolymers thereof. Further polymers suitable for use in the absorbent core include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthum gum, locust bean gum, and the like. Mixtures of natural and wholly or partially synthetic absorbent polymers can also be useful. Processes for preparing synthetic, absorbent gelling polymers are disclosed in U.S. Patent No. 4,076,663, issued to Masuda et al.*,* and U.S. Patent No. 4,286,082, issued to Tsubakimoto et al.

The high-absorbency material may be in a variety of geometric forms. It is desired that the high-absorbency material be in the form of discrete particles. However, the high-absorbency material may also be in the form of fibers, flakes, rods, spheres, needles, or the like. Often, the high-absorbency material is present in the absorbent core (50) in an amount of from about 5 to about 100 weight percent based on total weight of the absorbent core (50).

Referring again to FIG. 9, illustrated is a version of a diaper (40) in its generally flat-out or pre-activated state. The liner composite (30) is associated with and superposed on the outer cover (48) to thereby form the periphery (52) of the diaper (40). The periphery (52) defines an outer perimeter or edge(s) of the diaper (40). The periphery (52) generally includes longitudinal side edges (54) and lateral end edges (56). In each of the illustrated versions, the liner composite (30) includes a fluid pervious liner material (32) having a bodyfacing or upper surface, (36) and an opposing or lower surface (38). The liner composite (30) also includes a topographical surge (34) connected or otherwise associated with portions of the lower surface (38) of the liner material (32).

### TEST METHODS

### Procedure for Measuring Amplitude and Wavelength of a Topographical Surface

Amplitude and wavelength as described herein can be measured via optics, profilometery, or other imaging techniques. A preferred embodiment of a method would be the use of non-contact laser profilometery. The sample can be scanned at various resolutions/spacing in the X-Y-Z directions ranging from 200 mm to 0.001 mm. In the preferred embodiment, the scanner range would be about 10 um. Scanning should be such that a sufficient number of amplitude/wavelength ranges are scanned for measurements. The scanned data can be represented as a point-cloud ASCII format or any other means of format. Additional, the data can be transformed as necessary from the range of point-cloud raw data to completed surfaced data that could be exported to a CAD system or any other high-end surface format.

The measurements of amplitude and wavelength can be performed via various analysis programs. A preferred embodiment would be commercially available software such as Raindrop Geomagic's Studio and Qualify programs. Within the analysis programs, measurements of amplitude and wavelength can be preformed via "virtual" calipers or other measurement tools. For example, to measure amplitude (A in Figure 8), the maximum and minimum locations are selected using the caliper tool, and then the measurement is recorded. The recording of the data can be via the software (or macro, batch process) or manually.

### Procedure for Determining Material Caliper (thickness):

The caliper of a material is a measure of thickness and is measured at 0.05 psi (3.5 g/cm².) with a STARRET® bulk tester, in units of millimeters. Samples are cut into 4 inch by 4 inch (10.2 cm by10.2 cm) squares, five samples are tested, and the results averaged.

## Claims

1. A liner composite (30) comprising:
a fluid pervious liner (32) having a bodyfacing surface (36) and a lower surface (38);
a topographical surge material (34) having a bodyfacing surface (33), and a plurality of topographical features, at least a portion of the bodyfacing surface (33) of the topographical surge material (34) in facing relationship with at least a portion of the lower surface (38) of the fluid pervious liner (32);
wherein at least a portion of the topographical features are imparted onto the bodyfacing surface (36) of the fluid pervious liner (32), and a plurality of the topographical features imparted onto the bodyfacing surface (36) of the fluid pervious liner (32) have an amplitude of greater than about 2.5 mm; and
at least a portion of the fluid pervious liner (32) is a coapertured with the topographical surge material (34);
**characterised in that** the topographical surge material (34) is a meltblown or spunbond web of synthetic fibers, or a bonded-carded-web.

2. A disposable absorbent article comprising:
a liquid impervious backing sheet;
a fluid pervious liner (32) having a bodyfacing surface (36) and a lower surface (38);
an absorbent core disposed between the fluid pervious bodyside liner (32) and the liquid impervious backing sheet; and
a topographical surge material (34) having a plurality of topographical features, the topographical surge material (34) disposed between the absorbent core and the fluid pervious bodyside liner (32);
wherein at least a portion of the topographical features are imparted onto the bodyfacing surface (36) of the fluid pervious liner (32), and a plurality of the topographical features imparted onto the bodyfacing surface (36) of the fluid pervious liner (32) have an amplitude of greater than about 2.5 mm; and
at least a portion of the fluid pervious liner (32) is a coapertured with the topographical surge material (34);
**characterised in that** the topographical surge material (34) is a meltblown or spunbond web of synthetic fibers, or a bonded-carded-web.

3. The liner composite (30) or disposable absorbent article of claims 1 or 2, wherein a plurality of the topographical features imparted onto the bodyfacing surface (36) of the fluid pervious liner (32) have an amplitude of greater than about 5 mm.

4. The liner composite (30) or disposable absorbent article of claims 1 or 2, wherein a plurality of the topographical features imparted onto the bodyfacing surface (36) of the fluid pervious liner (32) have an amplitude of greater than about 15 mm.

5. The liner composite (30) or disposable absorbent article of claims 1 or 2, wherein at least a portion of the topographical surge material (34) is attached to at least a portion of the fluid pervious liner (32) by adhesive, cohesive, thermal, pressure, or ultrasonic bonds.

6. The liner composite (30) or disposable absorbent article of claims 1 or 2, wherein the fluid pervious liner (32) has a caliper of less than about 4 mm.

7. The liner composite (30) or disposable absorbent article of claims 1 or 2, wherein the fluid pervious liner (32) comprises a plurality of apertures (39).

8. The liner composite (30) or disposable absorbent article of claims 1 or 2, wherein at least a portion of the plurality of apertures (39) have at least one dimension greater than about 0.25 millimeters.

9. The liner composite (30) or disposable absorbent article of claims 1 or 2, wherein at least a portion of the plurality of apertures (39) have at least one dimension greater than about 0.5 millimeters.

10. The liner composite (30) or disposable absorbent article of claims 1 or 2, wherein at least a portion of the plurality of apertures (39) have at least one dimension greater than about 1.0 millimeters.

11. The liner composite (30) or disposable absorbent article of claims 1 or 2, wherein a plurality of the topographical features imparted onto the bodyfacing surface (36) of the fluid pervious liner (32) have a wavelength of between about 5 mm and about 25 mm.

12. The liner composite (30) or disposable absorbent article of claims 1 or 2, wherein the fluid pervious liner (32) has an area, the topographical surge material (34) has an area, and the ratio of the area of the topographical surge material (34) to the area of the fluid pervious liner (32) is no greater than about 0.75.

13. The liner composite (30) or disposable absorbent article of claim 12, wherein the ratio of the area of the topographical surge material (34) to the area of the fluid pervious liner (32) is no greater than about 0.5.

14. The liner composite (30) or disposable absorbent article of claim 12, wherein the ratio of the area of the topographical surge material (34) to the area of the fluid pervious liner (32) is no greater than about 0.1.

15. The liner composite (30) or disposable absorbent article of claims 1 or 2, further comprising a second surge material in facing relationship with at least a portion of the lower surface (38) of the fluid pervious liner (32), wherein the topographical surge material (34) is dissimilar from the second surge material.

16. The disposable absorbent article of claim 2, wherein the topographical surge material (34) has a basis weight of greater than about 30 gsm.

17. The disposable absorbent article of claim 2, wherein the topographical surge material (34) has a basis weight of at greater than about 50 gsm.

18. The disposable absorbent article of claim 2, wherein the topographical surge material (34) comprises a nonwoven material having a basis weight of at greater than about 30 gsm.

## Patentansprüche

1. Auskleidung-Komposit-Material (30), umfassend:
eine flüssigkeitsdurchlässige Auskleidung (32) mit einer zum Körper weisenden Fläche (36) und einer unteren Fläche (38) ;
ein topographisches Schwallmaterial (34) mit einer zum Körper weisenden Fläche (33) und einer Vielzahl von topographischen Merkmalen, wobei mindestens ein Teil der zum Körper weisenden Fläche (33) des topographischen Schwallmaterials (34) mindestens einem Teil der unteren Fläche (38) der flüssigkeitsdurchlässigen Auskleidung (32) gegenüberliegend ist;
worin mindestens ein Teil der topographischen Merkmale auf der zum Körper weisenden Fläche (36) der flüssigkeitsdurchlässigen Auskleidung (32) aufgebracht ist, und eine Vielzahl von topographischen Merkmalen, die auf der zum Körper weisenden Fläche (36) der flüssigkeitsdurchlässigen Auskleidung (32) aufgebracht sind, eine Amplitude von mehr als 2,5 mm aufweisen; und
mindestens ein Teil der flüssigkeitsdurchlässigen Auskleidung (32) mit den gleichen Öffnungen versehen ist wie das topographische Schwallmaterial (34);
**dadurch gekennzeichnet, dass** das topographische Schwallmaterial (34) eine schmelzgeblasene oder spinngebundene Bahn synthetischer Fasern oder eine gebondetekardierte Bahn ist.

2. Absorptionsfähiger Einwegartikel, umfassend:
eine flüssigkeitsdurchlässige Trägerschicht;
eine flüssigkeitsdurchlässige Auskleidung (32) mit einer zum Körper weisenden Fläche (36) und einer unteren Fläche (38) ;
einen absorbierenden Kern, der zwischen der flüssigkeitsdurchlässigen Körperseitenauskleidung (32) und der flüssigkeitsundurchlässigen Trägerschicht angeordnet ist; und
ein topographisches Schwallmaterial (34) mit einer Vielzahl von topographischen Merkmalen, wobei das topographische Schwallmaterial (34) zwischen dem absorbierenden Kern und der flüssigkeitsdurchlässigen Körperseitenauskleidung (32) angeordnet ist;
worin mindestens ein Teil der topographischen Merkmale auf der zum Körper weisenden Fläche (36) der flüssigkeitsdurchlässigen Auskleidung (32) aufgebracht ist, und eine Vielzahl von topographischen Merkmalen, die auf der zum Körper weisenden Fläche (36) der flüssigkeitsdurchlässigen Auskleidung (32) aufgebracht sind, eine Amplitude von mehr als 2,5 mm aufweisen; und
mindestens ein Teil der flüssigkeitsdurchlässigen Auskleidung (32) mit den gleichen Öffnungen versehen ist wie das topographische Schwallmaterial (34);
**dadurch gekennzeichnet, dass** das topographische Schwallmaterial (34) eine schmelzgeblasene oder spinngebundene Bahn synthetischer Fasern oder eine gebondetekardierte Bahn ist.

3. Auskleidung-Komposit-Material (30) oder absorbierender Einwegartikel nach Ansprüchen 1 oder 2, worin die Vielzahl von topographischen Merkmalen, die auf der körperseitigen Fläche (36) der flüssigkeitsdurchlässigen Auskleidung (32) aufgetragen ist, eine Amplitude von mehr als 5 mm aufweisen.

4. Auskleidung-Komposit-Material (30) oder absorbierender Einwegartikel nach Ansprüchen 1 oder 2, worin die Vielzahl von topographischen Merkmalen, die auf der körperseitigen Fläche (36) der flüssigkeitsdurchlässigen Auskleidung (32) aufgetragen ist, eine Amplitude von mehr als 15 mm aufweisen.

5. Auskleidung-Komposit-Material (30) oder absorbierender Einwegartikel nach Ansprüchen 1 oder 2, worin mindestens ein Teil des topographischen Schwallmaterials (34) an mindestens einem Teil der flüssigkeitsdurchlässigen Auskleidung (32) durch Klebe-, kohäsive, thermische, Druck- oder Ultraschallbindungen befestigt ist.

6. Auskleidung-Komposit-Material (30) oder absorbierender Einwegartikel nach Ansprüchen 1 oder 2, worin die flüssigkeitsdurchlässige Auskleidung (32) eine Dicke von weniger als etwa 4 mm aufweist.

7. Auskleidung-Komposit-Material (30) oder absorbierender Einwegartikel nach Ansprüchen 1 oder 2, worin die flüssigkeitsdurchlässige Auskleidung (32) eine Vielzahl von Öffnungen (39) umfasst.

8. Auskleidung-Komposit-Material (30) oder absorbierender Einwegartikel nach Ansprüchen 1 oder 2, worin mindestens ein Teil der Vielzahl von Öffnungen (39) mindestens eine Dimension größer als etwa 0,25 Millimeter aufweist.

9. Auskleidung-Komposit-Material (30) oder absorbierender Einwegartikel nach Ansprüchen 1 oder 2, worin mindestens ein Teil der Vielzahl von Öffnungen (39) mindestens eine Dimension größer als etwa 0,5 Millimeter aufweist.

10. Auskleidung-Komposit-Material (30) oder absorbierender Einwegartikel nach Ansprüchen 1 oder 2, worin mindestens ein Teil der Vielzahl von Öffnungen (39) mindestens eine Dimension größer als etwa 1,0 Millimeter aufweist.

11. Auskleidung-Komposit-Material (30) oder absorbierender Einwegartikel nach Ansprüchen 1 oder 2, worin die Vielzahl von topographischen Merkmalen, die auf der körperseitigen Fläche (36) der flüssigkeitsdurchlässigen Auskleidung (32) aufgetragen ist, eine Wellenlänge von zwischen etwa 5 mm und etwa 25 mm aufweisen.

12. Auskleidung-Komposit-Material (30) oder absorbierender Einwegartikel nach Ansprüchen 1 oder 2, worin die flüssigkeitsdurchlässige Auskleidung (32) einen Bereich aufweist, das topographische Schwallmaterial (34) einen Bereich aufweist und das Verhältnis des Bereichs des topographischen Schwallmaterials (34) zum Bereich der flüssigkeitsdurchlässigen Auskleidung (32) nicht größer ist als etwa 0,75.

13. Auskleidung-Komposit-Material (30) oder absorbierender Einwegartikel nach Anspruch 12, worin das Verhältnis des Bereichs des topographischen Schwallmaterials (34) zum Bereich der flüssigkeitsdurchlässigen Auskleidung (32) nicht größer ist als etwa 0,5.

14. Auskleidung-Komposit-Material (30) oder absorbierender Einwegartikel nach Anspruch 12, worin das Verhältnis des Bereichs des topographischen Schwallmaterials (34) zum Bereich der flüssigkeitsdurchlässigen Auskleidung (32) nicht größer ist als etwa 0,1.

15. Auskleidung-Komposit-Material (30) oder absorbierender Einwegartikel nach Ansprüchen 1 oder 2, ferner umfassend ein zweites Schwallmaterial, das mindestens einem Teil der unteren Fläche (38) der flüssigkeitsdurchlässigen Auskleidung (32) gegenüberliegend ist, worin das topographische Schwallmaterial (34) sich vom zweiten Schwallmaterial unterscheidet.

16. Absorbierender Einwegartikel nach Anspruch 2, worin das topographische Schwallmaterial (34) ein Flächengewicht von mehr als etwa 30 g/m² aufweist.

17. Absorbierender Einwegartikel nach Anspruch 2, worin das topographische Schwallmaterial (34) ein Flächengewicht von mehr als etwa 50 g/m² aufweist.

18. Absorbierender Einwegartikel nach Anspruch 2, worin das topographische Schwallmaterial (34) ein Vliesmaterial umfasst, das ein Flächengewicht von mehr als 30 g/m² aufweist.

## Revendications

1. Revêtement composite (30) comprenant :
un revêtement perméable au fluide (32) présentant une surface tournée vers le corps (36) et une surface inférieure (38) ;
un matériau de pointe topographique (34) présentant une surface tournée vers le corps (33), et une pluralité de caractéristiques topographiques, au moins une partie de la surface tournée vers le corps (33) du matériau de pointe topographique (34) en relation d'opposition avec au moins une partie de la surface inférieure (38) du revêtement perméable au fluide (32) ;
dans lequel au moins une partie des caractéristiques topographiques sont transmises à la surface tournée vers le corps (36) du revêtement perméable au fluide (32), et une pluralité des caractéristiques topographiques conférées à la surface tournée vers le corps (36) du revêtement perméable au fluide (32) ont une amplitude supérieure à environ 2,5 mm ; et
au moins une partie du revêtement perméable au fluide (32) est perforée coaxialement avec le matériau de pointe topographique (34) ;
**caractérisé en ce que** le matériau de pointe topographique (34) est une bande obtenue par extrusion soufflage ou filée-liée de fibres synthétiques ou une bande cardée liée.

2. Article absorbant jetable comprenant :
une feuille de support imperméable aux liquides ;
un revêtement perméable au fluide (32) présentant une surface tournée vers le corps (36) et une surface inférieure (38) ;
un noyau absorbant disposé entre le revêtement à doublure côté corps perméable aux fluides (32) et la feuille de support imperméable aux liquides ; et
un matériau de pointe topographique (34) ayant une pluralité de caractéristiques topographiques, le matériau de pointe topographique (34) disposé entre le noyau absorbant et le revêtement à doublure côté corps perméable aux fluides (32) ;
dans lequel au moins une partie des caractéristiques topographiques sont transmises à la surface tournée vers le corps (36) du revêtement perméable au fluide (32), et une pluralité des caractéristiques topographiques conférées à la surface tournée vers le corps (36) du revêtement perméable au fluide (32) ont une amplitude supérieure à environ 2,5 mm ; et
au moins une partie du revêtement perméable au fluide (32) est perforée coaxialement avec le matériau de pointe topographique (34) ;
**caractérisé en ce que** le matériau de pointe topographique (34) est une bande obtenue par extrusion soufflage ou filée-liée de fibres synthétiques ou une bande cardée liée.

3. Revêtement composite (30) ou article absorbant jetable selon les revendications 1 ou 2, dans lequel une pluralité des caractéristiques topographiques conférées à la surface tournée vers le corps (36) du revêtement perméable au fluide (32) ont une amplitude supérieure à environ 5 mm.

4. Revêtement composite (30) ou article absorbant jetable selon les revendications 1 ou 2, dans lequel une pluralité des caractéristiques topographiques conférées à la surface tournée vers le corps (36) du revêtement perméable au fluide (32) ont une amplitude supérieure à environ 15 mm.

5. Revêtement composite (30) ou article absorbant jetable selon les revendications 1 ou 2, dans lequel au moins une partie du matériau de pointe topographique (34) est attachée à au moins une partie du revêtement perméable au fluide (32) par liaisons adhésives, cohésives, thermiques, par pression ou par ultrasons.

6. Revêtement composite (30) ou article absorbant jetable selon les revendications 1 ou 2, dans lequel le revêtement perméable au fluide (32) a une épaisseur inférieure à environ 4 mm.

7. Revêtement composite (30) ou article absorbant jetable selon les revendications 1 ou 2, dans lequel le revêtement perméable au fluide (32) comprend une pluralité d'ouvertures (39) .

8. Revêtement composite (30) ou article absorbant jetable selon les revendications 1 ou 2, dans lequel au moins une partie de la pluralité d'ouvertures (39) ont au moins une dimension supérieure à environ 0,25 millimètres.

9. Revêtement composite (30) ou article absorbant jetable selon les revendications 1 ou 2, dans lequel au moins une partie de la pluralité d'ouvertures (39) ont au moins une dimension supérieure à environ 0,5 millimètres.

10. Revêtement composite (30) ou article absorbant jetable selon les revendications 1 ou 2, dans lequel au moins une partie de la pluralité d'ouvertures (39) ont au moins une dimension supérieure à environ 1,0 millimètres.

11. Revêtement composite (30) ou article absorbant jetable selon les revendications 1 ou 2, dans lequel une pluralité des caractéristiques topographiques conférées à la surface tournée vers le corps (36) du revêtement perméable au fluide (32) ont une longueur d'onde comprise entre environ 5 mm et environ 25 mm.

12. Revêtement composite (30) ou article absorbant jetable selon les revendications 1 ou 2, dans lequel le revêtement perméable au fluide (32) comporte une zone, le matériau de pointe topographique (34) comporte une zone, et le rapport de la zone du matériau de pointe topographique (34) à la zone du revêtement perméable au fluide (32) n'est pas supérieur à environ 0,75.

13. Revêtement composite (30) ou article absorbant jetable selon la revendication 12, dans lequel le rapport de la zone du matériau de pointe topographique (34) à la zone du revêtement perméable au fluide (32) n'est pas supérieur à environ 0,5.

14. Revêtement composite (30) ou article absorbant jetable selon la revendication 12, dans lequel le rapport de la zone du matériau de pointe topographique (34) à la zone du revêtement perméable au fluide (32) n'est pas supérieur à environ 0,1.

15. Revêtement composite (30) ou article absorbant jetable selon les revendications 1 ou 2, comprenant en outre un deuxième matériau de pointe en relation d'opposition avec au moins une partie de la surface inférieure (38) du revêtement perméable au fluide (32), dans lequel le matériau de pointe topographique (34) est différent du deuxième matériau de pointe.

16. Article absorbant jetable selon la revendication 2, dans lequel le matériau de pointe topographique (34) a un poids de base supérieur à environ 30 grammes par mètre carré.

17. Article absorbant jetable selon la revendication 2, dans lequel le matériau de pointe topographique (34) a un poids de base supérieur à environ 50 grammes par mètre carré.

18. Article absorbant jetable selon la revendication 2, dans lequel le matériau de pointe topographique (34) comprend un matériau non tissé ayant un poids de base supérieur à environ 30 grammes par mètre carré.
